# EUROPEAN PATENT SPECIFICATION

(11) **EP 3 148 468 B1**
(45) Date of publication and mention of the grant of the patent: **04.07.2018**
(21) Application number: 15726084.5
(22) Date of filing: 25.05.2015
(51) Int. Cl.: A61B 18/20

(54) **APPARATUS FOR STIMULATION AND/OR TREATMENT OF ANATOMICAL TISSUES**
VORRICHTUNG ZUR STIMULATION UND/ODER BEHANDLUNG VON ANATOMISCHEN GEWEBEN
APPAREIL POUR LA STIMULATION ET/OU LE TRAITEMENT DE TISSUS ANATOMIQUES

(30) Priority: 27.05.2014 IT BO20140310
(43) Date of publication of application: 05.04.2017
(73) Proprietor: Tecnologica S.A.S. di Venella Salvatore & C., 61122 Pesaro (IT)
(72) Inventor: VANELLA, Salvatore, 61025 Monteciccardo (IT)
(74) Representative: Baldi, Claudio
(86) International application number: PCT/EP2015/061488
(87) International publication number: WO 2015/181102

(56) References cited:
- WO-A1-2014/064074
- WO-A1-2014/064074
- US-A1- 2002 173 781
- US-A1- 2002 173 781
- US-A1- 2006 200 116
- US-A1- 2006 200 116
- US-A1- 2008 058 783
- US-A1- 2008 058 783
- US-A1- 2012 215 210
- US-A1- 2012 215 210
- US-A1- 2012 296 260
- US-A1- 2012 296 260

## Description

The present invention relates to an apparatus for the stimulation and/or treatment of anatomical tissues.

The use of laser emitters (laser is the English acronym of Light Amplification by Stimulated Emission of Radiation) with the purpose of acting (in some way) on tissues (human and/or animal) is known: in particular, this use is widespread in the medical (surgical) field, in the field of rehabilitation, physiotherapy, beauty care and also in so-called cosmetic medicine.

More recently, small apparatuses for private and home use, by means of which the user can self-administer directly and autonomously the treatment based on the use of the laser have become very widespread.

Reference is made to documents WO2014/064074 A1 and US2006/200116 A1. In this context, it is appropriate to note that the CEI EN 60825-1 safety standard is in force and regulates and restricts the use of laser devices: this standard requires the doctor and the patient to use adapted individual protection devices, such as for example goggles, to protect the eyes from damage that might occur if they were struck by a laser beam.

This standard, though being in force for at least a couple of decades, however has not served to avoid the occurrence every year of many accidents with consequent severe pathological effects on the eyes.

In practice, the negligence of specialized operators (who by being accustomed to working with these devices tend to underestimate their danger) and the ignorance of private individuals (owners of devices for private and home use, by means of which it is indeed possible to self-administer directly the treatment based on the use of the laser) causes a number of accidents that arise from the alignment of the laser beam with the eyes.

Delicate organs such as the eyes in fact suffer damage of various extents (even very severe and permanent, even up to blindness) if a laser beam strikes their surface, even if only for short periods.

The aim of the present invention is to solve the problems described above, proposing an apparatus for the stimulation and treatment of anatomical tissues that is provided with a protection and safety assembly for laser emitters that is at least suitable to reduce the risks of unintentional lesions produced by the laser on delicate tissues such as those of the eyes.

Within the scope of this aim, an object of the invention is to propose an apparatus provided with a protection and safety assembly for laser emitters that is suitable to protect the eyes of any users (both active, performing the treatment, and passive, receiving the treatment) from the unintentional impingement of the laser beam.

Another object of the invention is to propose an apparatus that allows to use laser emitters for the treatment and stimulation of anatomical tissues in compliance with the statutory provisions in force.

A further object of the present invention is to provide an apparatus for the stimulation and/or treatment of anatomical tissues that has low costs, is relatively simple to provide in practice and is safe in application.

The invention is defined in the appended claims. This aim and these objects are achieved by an apparatus for the stimulation and/or treatment of anatomical tissues, comprising at least one emitter of a laser beam, controlled by a respective driving circuit provided with a unit for the control and management of said at least one emitter, with a power source and with at least one element for interrupting said driving circuit, which constitutes an element for powering on and off the entire said apparatus, characterized in that it comprises a protection and safety assembly for said at least one emitter, said assembly being arranged along said driving circuit and comprising a proximity sensor, the sensitive surface of which is arranged proximate to the emission area of the laser beam and the detection direction of which is parallel to the emission direction of said laser beam, said assembly comprising at least one control and management device, driven by said sensor and suitable at least to limit the power supplied to said laser beam emitter when said sensitive surface is separated from the area to be treated by an air gap that corresponds to a distance that exceeds a preset value.

Further characteristics and advantages of the invention will become better apparent from the description of two preferred but not exclusive embodiments of the apparatus according to the invention, illustrated by way of non-limiting example in the accompanying drawings, wherein:
Figure 1 is an exploded perspective view of a stimulation apparatus provided with the protection and safety assembly for laser emitters according to the invention, in a first embodiment;
Figure 2 is a perspective view of a stimulation apparatus provided with the protection and safety assembly for laser emitters according to the invention, in a second embodiment;
Figure 3 is the functional electrical diagram of a possible protection and safety assembly for laser emitters according to the invention.

With reference to the figures, the reference numeral 1 designates generally an apparatus for the stimulation and/or treatment of anatomical tissues.

The apparatus 1 comprises at least one emitter 2 of a laser beam, which is controlled by a respective driving circuit 3.

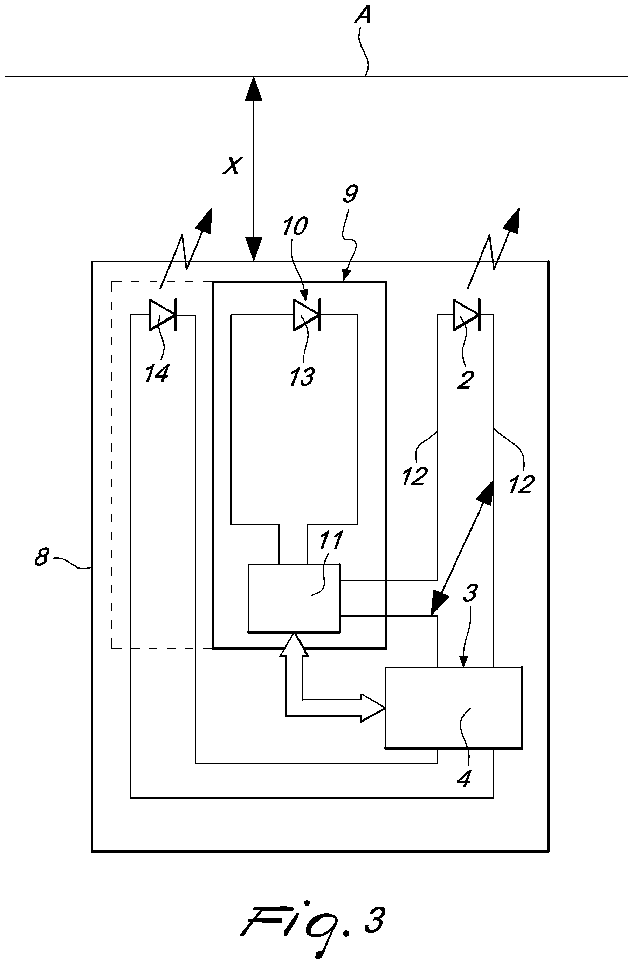

The driving circuit 3 in turn is provided with a unit 4 for the control and management of the at least one emitter 2, with a power source and with at least one element for interrupting the circuit 3, which constitutes a power on/off element for the entire apparatus 1 (in Figure 3, the power on/off element is to be considered included in the block that represents the control and management unit 4).

The power source is not shown in the accompanying figures, can be external or internal to the apparatus 1 (although usually these are batteries located within the apparatus 1) and is preset to supply electric power to all the components.

It is useful to specify right now that the protective scope claimed herein comprises apparatuses 1 of any type, designed in any case to act in some way on anatomical tissues (human and/or animal), for their stimulation and/or treatment. For example, mention is made of the possibility to use the apparatuses 1 (for professional or private use) in a medical (surgical) field, in the fields of rehabilitation, physiotherapy, beauty care and also in so-called cosmetic medicine.

Moreover, it should be noted that the apparatus 1 according to the invention can be provided with emitters 2 of any type, as a function of the specific requirements, without thereby abandoning the protective scope claimed herein.

According to a particular constructive solution, the apparatus 1 for stimulating tissues comprises a boxlike body 1a provided with an internal cavity 1b.

The cavity 1b is suitable to accommodate the control and management unit 4 and the driving circuit 3.

The emitter 2 is usually a single emitter, but it is specified right now that although the constructive solution that provides a single emitter 2 can be considered particularly effective, the adoption of two or more emitters 2, the operation of which may be synchronous, combined and/or independent, is not excluded; the control and management unit 4 is preset to adjust the duration of the emission of the laser beam emitted through a window 5 of the boxlike body 1a (in particular a closure plug 6 thereof).

The emitter 2 is accommodated on a respective plate 7, so that its position is determined uniquely inside the boxlike body 1a in order to utilize a beam emitted at the correct focal distance from the surface of the tissue to be treated.

During normal operation (for the treatment and/or stimulation of anatomical tissues), the laser beam is at wavelengths comprised between 600 nm and 1100 nm, while the frequency of the emission pulses corresponds to values comprised between 100 Hz and 10,000 Hz.

The treatment times are comprised by way of example between 1 minute and 99 minutes.

The control and management unit 4 defines the operating setting of the emitter 2, defining the emission frequency and the duration of the emission time of the laser beam in order to obtain the effect of stimulating and sensitizing the affected tissues, or more generally obtain the desired treatment.

For example, the emission of the laser beam that corresponds to a high-frequency beam of the emission pulses can achieve a sensitizing effect on the tissues that are struck by it.

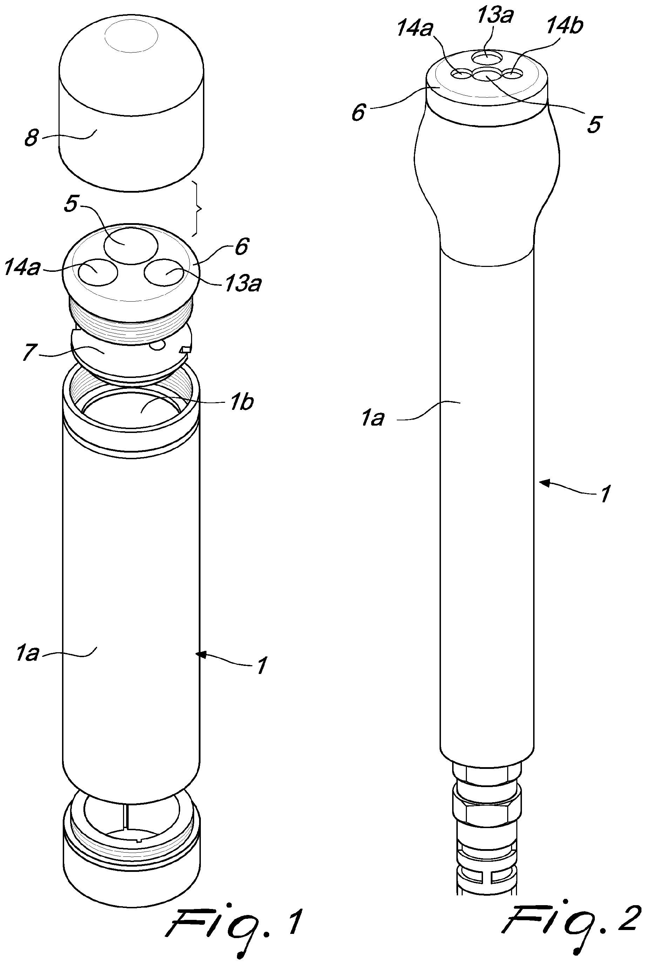

According to the constructive solutions shown in Figures 1 and 2, the boxlike body 1a is substantially cylindrical (the possibility to use bodies 1a having an elliptical, oval and/or polygonal cross-section, with appropriately rounded corners in order to avoid lesions of the tissues that come into contact with them, is not excluded).

The (lower and upper) closure elements of the boxlike body 1a are preferably of the hermetic type.

The closure plug 6 is substantially at least partially translucent and transparent with respect to the laser beam emitted by the emitter 2: the translucent portion of the plug 6 therefore constitutes the window 5 through which the laser beam can reach the tissue surface to be treated.

The transparent window 5, central part of the plug 6, can be a lens in order to concentrate or diffuse the emitted beam and this will enhance and amplify the effects of the treatment being performed.

It should be specified that the apparatus 1 can comprise (according to a constructive solution of particular practical interest, which does not limit in any way the possibility to have an apparatus 1 without this accessory) a suitable covering dome 8 that can be fitted on the top of the apparatus 1, substantially over the plug 6: the dome 8 behaves like a protection for the plug 6.

The possibility to provide a dome 8 that instead behaves like an optical filter and can, according to the requirements, diffuse the emitted light beam, concentrate it or filter it in terms of frequency.

The dome 8 is shaped like a hood that is shaped complementarily to the upper surface of the apparatus 1 and covers completely the plug 6 as well.

It is specified right now that the emitter 2 is preferably of the type of laser diodes, which generate a laser beam with a wavelength comprised substantially between 600 nm and 1100 nm: the possibility to adopt sources (emitters 2) of a different type with respect to laser diodes and/or to use beams emitted with wavelengths that differ even significantly from the indicated values (typical value of laser diodes used in the medical and cosmetic fields) is not excluded in any case.

Moreover, the possibility is not excluded to combine with the laser diodes also other sources (types of emitter) with the goal of combining the effects of beam emitted by the laser diode with the beam or beams emitted by another source or sources, amplifying and augmenting the effects on the surface to be treated.

According to the invention, the apparatus 1 comprises a protection and safety assembly 9 for the emitter 2 (or for the emitters 2); the assembly 9 is arranged along the driving circuit 3 and comprises a proximity sensor 10, the sensitive surface of which is arranged proximate to the emission area of the laser beam and the detection direction of which is parallel to the emission direction of said laser beam.

The assembly 9 comprises further at least one control and management device 11, which is driven by said sensor 10: the device 11 is suitable at least to limit the power supplied to the laser beam emitter 2 when the sensitive surface of the sensor 10 is separated from the area to be treated A by an air gap that corresponds to a distance X that is greater than a preset value.

According to a possible constructive solution, which is a nonlimiting example of the application of the invention, the control and management device 11 is constituted substantially by a switch that its preset to interrupt the driving circuit 3 when the sensitive service of the sensor 10 is separated from the area to be treated A by an air gap that corresponds to a distance X that exceeds a preset value.

According to a further constructive solution that is more efficient and its particularly indicated for use on the part of personnel that is not specialized and trained on laser emission sources, the control and management device 11 comprises means for adjusting the electrical values along the power supply cables 12 of the laser beam emitter 2.

These adjustment means, at a distance X that exceeds a preset value of the sensitive surface of the proximity sensor 10 from the area to be treated A, limit the electrical power supply values of the emitter 2 in order to limit accordingly the emitted laser beam, until a value that is lower than the value of regular operation and is not dangerous if it impinges on the eyes.

In practice, if the window 5 or the dome 8 are not close enough to the area to be treated A, the emitter 2 generates a flux of very low intensity that is not harmful for the eyes: even in case of accidental impingement of the luminous flux on the eyes, therefore, no consequence occurs.

In particular, if the emitters 2 are of the type of laser diodes, the beam emitted by each emitter 2, at a distance X that exceeds a preset value of the sensitive surface of the proximity sensor 10 from the area to be treated A, has a frequency comprised between 0.1 Hz and 10 Hz and an energy comprised between 0.2 µJ and 25 µJ.

More specifically, it is noted that in a preferred solution the beam emitted by each emitter 2, at a distance X that exceeds a preset value of the sensitive surface of the proximity sensor 10 from the area to be treated A, has a frequency equal to 1 Hz and an energy equal to 2.5 µJ.

It is appropriate to indicate that according to a constructive solution of unquestionable interest in practice and in application, the proximity sensor 10 comprises a photodiode 13, which is suitable to detect the photons that impinge on its sensitive surface and to send an electrical signal that is proportional to the extent of said detection to the control and management device 11.

The proximity sensor 10 further comprises a light source: the proximity to the surface to be treated A causes high reflection of the photons emitted by the light source, with a consequent high incidence thereof on the photodiode 13; a distance X greater than a preset value from the surface to be treated A instead causes a limited or even nil reflection of the photons emitted by the light source, with consequent minimum incidence thereof on the photodiode 13.

It is specified that the light source of the proximity sensor 10 can optionally be constituted by the laser beam emitter 2, which is usually preset to be activated, when the apparatus 1 is powered on, in operation with minimum emission intensity, which corresponds to a frequency comprised between 0.1 and 10 Hz and to an energy comprised between 0.2 and 25 µJ.

As an alternative, a constructive solution can be provided in which the light source is constituted by a specific LED that is designed to deliver a continuous light beam when the apparatus 1 is on (regardless of whether the emitter 2 is emitting the laser beam).

Usefully, the apparatus 1 according to the invention can comprise at least one luminous indicator 14 of the emission of the laser beam: said laser beam is in fact not in the visible spectrum and therefore the user is unable to identify its correct operation without an appropriate indicator 14.

For example, the function of luminous indicator 14 of the emission of the laser beam can be performed by the LED introduced above, which is preset to deliver a continuous light beam and therefore to also perform the function of a light source.

In particular, the apparatus 1 might comprise at least one first luminous indicator 14 for indicating the emission of the minimum-intensity beam, which is not dangerous for the eyes, and at least one second luminous indicator 14 for indicating the emission of the beam with an intensity suitable for the stimulation treatment, which is dangerous for the eyes.

Preferably, the control and management device 11 is of the type of a chip, integrated circuit, arranged along the driving circuit 3.

The CEI EN 60825-1 safety standard for the use of lasers in the medical field requires the physician and the patient to use adapted protective solutions (for example goggles, as observed in known solutions) in order to protect the eyes against damage that might occur if struck by a laser beam.

By avoiding the adoption of protective goggles (which is often found to be scarcely practical, so much that it is neglected by distracted or scarcely professional users), the present invention proposes an assembly 9 that is provided with a "proximity sensor" 10 that is capable of detecting automatically, once the apparatus 1 has been turned on, the presence of a surface A within a certain distance, termed nominal range or sensitive field, from the "sensitive side" of the sensor 10.

This evidently allows to use emitters 2 to perform treatment or stimulation of anatomical tissues in full compliance with the statutory provisions in force.

The detection distance within which the proximity sensor 10 recognizes, according to a preferred constructive solution, the presence of a surface A, can be adjusted to 2-3-5 centimeters, since therapeutic, surgical and cosmetic handpieces are used by the operator in contact, or almost in contact, with the surface to be treated A.

In particular, when correct programming of the operation of the apparatus 1 has been performed and once the emission command has been given, if the distance X detected between the surface to be treated A and the emitter 2 (and therefore also of the sensor 10) contained in the laser handpiece is equal to or lower than the preset distance, the emitter 2 emits its beam at the programmed frequency and power values.

Vice versa, if the detected distance X is greater than the preset distance, the emitter 2 delivers a pulse equal to 1 Hz and 2.5 µJ of power, which is entirely harmless for the human eye.

Therefore, only when the emitter 2 (and the proximity sensor 10) controlled by the assembly 9 according to the invention is moved close to the surface to be treated does the emitter deliver at the programmed values.

When instead the emitter 2 (and the proximity sensor 10) is spaced from the surface to be treated A, the emitter 2 interrupts the emission of the laser beam at the programmed values and enters the safe mode of operation.

The proximity sensor 10 is preferably provided by means of a photodiode 13 that is arranged within the body 1a, substantially adjacent to the emitter 2. The photodiode 13 is interfaced continuously (by means of the device 11) with the unit 4 that controls the emitter 2.

When the user presses the power-on control of the apparatus 1, the emitter 2 emits a laser beam at 1 Hz.

By then moving the dome 8 toward the surface to be treated A (even in the case of a skin with phototype VI) the photodiode 13, which operates like an optical sensor, detects a certain quantity of photons emitted by the emitter 2 and reflected by the surface A being considered. If this quantity exceeds a given threshold, the unit 4 automatically activates the delivery of the laser beam at the programmed power and frequency values.

When instead the apparatus 1 is moved away from the surface to be treated A, the unit 4 that controls the generation of the pulses returns to the initial state of emission at 1 Hz and 2.5 µJ in power, ready to pass again to full emission at the set frequency when the sensor 10 detects a sufficient value of reflected light.

The use of the assembly 9 increases safety whenever a laser apparatus is forgotten on or is pointed unintentionally toward the eyes.

The assembly 9 can be applied to the various laser handpieces that are commercially available and is particularly useful for lasers for personal use.

Advantageously, the protection and safety assembly 9 for emitters 2, in particular for apparatuses 1 for the stimulation and treatment of anatomical tissues, is suitable to reduce to a minimum and potentially even eliminate the risks of unintentional injuries produced by the laser on delicate tissues such as those of the eyes.

Positively, the assembly 9 is adapted to protect the eyes of any users (both active ones, performing the treatment, and passive ones, receiving the treatment) from the unintentional impingement of the laser beam without requiring the use of screens (goggles and/or actual screens), thus rendering the apparatus 1 provided with the assembly 9 inherently safe.

Positively, the assembly 9 makes it impossible for the apparatus 1 that controls it to emit a laser beam without said apparatus being in use, even if it is left on unintentionally.

The invention thus conceived is susceptible of numerous modifications and variations, all of which are within the scope of the appended claims; all the details may further be replaced with other technically equivalent elements.

For example, and as can be deduced from the embodiment proposed merely by way of example in Figure 1, the closure plug 6 might comprise specific openings 13a and 14a intended for the passage of light toward the photodiode 13 and toward the luminous indicator 14 (if the apparatus 1 has only one), or might be made entirely of transparent material.

As an alternative, in a different embodiment, which is proposed without intending to limit the application of the invention in Figure 2, and in which the apparatus 1 is provided with two luminous indicators 14 (capable of also performing the function of light pointers), the closure plug 6 can also comprise, in addition to the openings 13a and 14a intended respectively for passage of light toward the photodiode 13 and toward the first luminous indicator 14, an opening 15b for the passage of the light toward the second indicator 14.

In the exemplary embodiments shown, individual characteristics, given in relation to specific examples, may actually be interchanged with other different characteristics that exist in other exemplary embodiments.

In practice, the materials used, as well as the dimensions, may be any according to requirements and to the state of the art.

The disclosures in Italian Patent Application No. BO2014A000310 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An apparatus for the stimulation and/or treatment of anatomical tissues, comprising at least one emitter (2) of a laser beam, controlled by a respective driving circuit (3) provided with a unit (4) for the control and management of said at least one emitter (2), with a power source and with at least one element for interrupting said driving circuit (3), which constitutes an element for powering on and off the entire said apparatus (1), the apparatus comprising a protection and safety assembly (9) for said at least one emitter (2), said assembly (9) being configured to be arranged along said driving circuit (3) and comprising a proximity sensor (10), the sensitive surface of which is arranged proximate to the emission area of the laser beam and the detection direction of which is parallel to the emission direction of said laser beam, said assembly (9) comprising at least one control and management device (11), driven by said sensor (10) and configured to limit the power supplied to said laser beam emitter (2) when said sensitive surface is separated from the area to be treated (A) by an air gap that corresponds to a distance (X) from the sensitive surface of said proximity sensor (10) to the area to be treated exceeding a preset value, wherein
said control and management device (11) comprises means for adjusting the electrical values along the power supply cables (12) of said beam emitter (2), said means being configured to limit the electrical power supply values of the emitter (2) in order to limit emission of the laser beam to a value that is lower than its normal operating value, if the distance from the sensitive surface of said proximity sensor (10) to the area to be treated (A) is detected to be greater than said preset value, wherein said emitter (2) is of the type of laser diodes, **characterized in that** said emitter (2) is configured to emit a beam having a frequency in the range between 0.1 and 10 Hz and an energy in the range between 0.2 and 25 µJ, if the distance (X) from the sensitive surface of said proximity sensor (10) to the area to be treated is detected to be greater than the preset value.

2. The apparatus according to claim 1, **characterized in that** said emitter (2) is configured to emit a beam having a frequency of 1 Hz and an energy of 2.5 µJ, if the distance (X) of the sensitive surface of said proximity sensor (10) to the area to be treated is detected to be greater than the preset value.

3. The apparatus according to claim 1, **characterized in that** said proximity sensor (10) comprises a light source and a photodiode (13), which is adapted to detect the photons that are incident on its sensitive surface and to send an electrical signal that is proportional to the extent of said detection to said control and management device (11), the proximity of the apparatus to the surface to be treated (A) producing a high reflection of the photons emitted by the light source, with a consequent high incidence thereof on said photodiode (13), wherein the distance (X) exceeding said preset value produces a low and even nil reflection of the photons emitted by the light source, with consequent minimum incidence thereof on said photodiode (13).

4. The apparatus according to claim 3, **characterized in that** said light source of said proximity sensor (10) is formed by said emitter (2) of a laser beam, wherein, if the apparatus is powered on, said emitter (2) is preset for activation in operation with minimum emission intensity, which corresponds to the frequency range between 0.1 and 10 Hz and the energy range between 0.2 and 25 µJ.

5. The apparatus according to one or more of the preceding claims, **characterized in that** it comprises at least one luminous indicator (14) of the emission of the laser beam.

6. The apparatus according to claim 5, **characterized in that** it comprises at least one first luminous indicator (14) indicating the emission of the beam with minimum intensity and at least one second luminous indicator (14) for indicating the emission of the beam with an intensity suitable for the stimulation treatment.

7. The apparatus according to one or more of the preceding claims, **characterized in that** said control and management device (11) is of the type of a chip or integrated circuit arranged along said driving circuit (3).

## Patentansprüche

1. Gerät zur Stimulierung und/oder Behandlung von anatomischem Gewebe, umfassend wenigstens eine Laserstrahl emittierende Einheit (2), die von einer jeweiligen Treiberschaltung (3) gesteuert wird, die mit einer Steuer- und Regeleinheit (4) der wenigstens einen emittierenden Einheit (2), einer Stromquelle und wenigstens einem Element zum Unterbrechen der Treiberschaltung (3), versehen ist, welches ein Element zum Ein-/Ausschalten des Geräts (1) bildet,
wobei das Gerät eine Schutz- und Sicherheitseinrichtung (9) für die wenigstens eine emittierende Einheit (2) umfasst, wobei die Einrichtung (9) ausgebildet ist, um entlang der Treiberschaltung (3) angeordnet zu werden und einen Näherungssensor (10) mit einer sensiblen Fläche umfasst, die in der Nähe der den Laserstrahl emittierenden Fläche angeordnet ist und deren Erfassungsrichtung parallel zur Emissionsrichtung des Laserstrahls verläuft, wobei die Einrichtung (9) wenigstens eine Steuer- und Regelvorrichtung (11) umfasst, die von dem Sensor (10) betätigt wird und ausgebildet ist, um die Leistung, die von der den Laserstrahl emittierenden Einheit (2) bereitgestellt wird, zu begrenzen, wenn die sensible Fläche von der zu behandelnden Fläche (A) durch einen Luftraum getrennt ist, der einem Abstand (X) zwischen der sensiblen Fläche des Näherungssensors (10) und der zu behandelnden Fläche (A) entspricht, einen zuvor festgelegten Wert überschreitet,
wobei
die Steuer- und Regelvorrichtung (11) Mittel zur Einstellung der elektrischen Größen längs der Versorgungskabel (12) der den Laserstrahl emittierenden Einheit (2) umfasst, wobei die Mittel ausgebildet sind, um die elektrischen Werte der Stromversorgung der emittierenden Einheit (2) zu begrenzen, um die Emission des Laserstrahls auf einen Wert zu begrenzen, der unterhalb des normalen Betriebswertes liegt, wenn der zwischen der sensiblen Fläche des Näherungssensors (10) und der zu behandelnden Fläche (A) gemessene Abstand größer als der zuvor festgelegte Wert ist,
wobei die emittierende Einheit (2) vom Laserdiodentyp ist,
**dadurch gekennzeichnet, dass**
die emittierende Einheit (2) ausgebildet ist, um einen Strahl mit einer Frequenz zwischen 0,1 und 10 Hz und einer Energie zwischen 0,2 und 25 µJ zu emittieren, wenn der zwischen der sensiblen Fläche des Näherungssensors (10) und der zu behandelnden Fläche gemessene Abstand (X) größer als der zuvor festgelegte Wert ist.

2. Gerät nach Anspruch 1, **dadurch gekennzeichnet, dass** die emittierende Einheit (2) ausgebildet ist, um einen Strahl mit einer Frequenz von 1 Hz und einer Energie zwischen von 2,5 µJ zu emittieren, wenn der zwischen der sensiblen Fläche des Näherungssensors (10) und der zu behandelnden Fläche gemessene Abstand (X) größer als der zuvor festgelegte Wert ist.

3. Gerät nach Anspruch 1, **dadurch gekennzeichnet, dass** der Näherungssensor (10) eine Lichtquelle und eine Fotodiode (13) umfasst, die zur Erfassung der auf seine sensible Fläche eintreffenden Fotonen und zur Übermittlung eines zur gemessenen Größe proportionalen elektrischen Signals an die Steuer- und Regelvorrichtung (11) geeignet ist, wobei die Nähe des Geräts zu der zu behandelnden Fläche (A) eine erhöhte Reflexion der von der Lichtquelle emittierten Fotonen und demzufolge einen erhöhten Einfall derselben auf die Fotodiode (13) bewirkt, wobei ein Abstand (X), der größer als der zuvor festgelegte Wert ist, eine geringe oder auch keine Reflexion der von der Lichtquelle abgegebenen Fotonen und demzufolge einen minimalen Einfall derselben auf die Fotodiode (13) bewirkt.

4. Gerät nach Anspruch 3, **dadurch gekennzeichnet, dass** die Lichtquelle des Näherungssensors (10) aus der einen Laserstrahl emittierenden Einheit (2) besteht, wobei die emittierende Einheit (2) bei eingeschaltetem Gerät auf die Ansteuerung bei Betrieb mit minimaler Emissionsintensität voreingestellt ist, die einer Frequenz zwischen 0,1 und 10 Hz und einer Energie zwischen 0,2 und 25 µJ entspricht.

5. Gerät nach einem oder mehreren der vorstehenden Ansprüche, **dadurch gekennzeichnet, dass** es wenigstens eine Leuchtanzeige (14) des emittierten Laserstrahls umfasst.

6. Gerät nach Anspruch 5, **dadurch gekennzeichnet**, es wenigstens eine erste Leuchtanzeige (14) zur Anzeige des mit minimaler Intensität emittierten Laserstrahls und wenigstens eine zweite Leuchtanzeige (14) zur Anzeige des mit einer für die Stimulierungsbehandlung geeigneten Intensität emittierten Laserstrahls umfasst.

7. Gerät nach einem oder mehreren der vorstehenden Ansprüche, **dadurch gekennzeichnet, dass** die Steuer- und Regelvorrichtung (11) vom Typ eines Chips oder einer integrierten Schaltung ist, die längs der Treiberschaltung (3). angeordnet ist.

## Revendications

1. Appareil de stimulation et/ou traitement de tissus anatomiques, comprenant au moins un émetteur (2) d'un rayon laser, contrôlé par un respectif circuit d'actionnement (3) muni d'une unité de commande et gestion (4) du dit au moins un émetteur (2), d'une source d'alimentation et d'au moins un élément d'interruption du dit circuit d'actionnement (3), constituant un organe d'allumage/extinction de tout l'appareil (1),
l'appareil comprenant un groupe (9) de protection et sécurité pour ledit au moins un émetteur (2), ledit groupe (9) étant configuré de manière à être disposé le long du dit circuit d'actionnement (3) et comprenant un capteur de proximité (10), ayant une surface sensible disposée à proximité de la zone d'émission du rayon laser et une direction de détection parallèle à la direction d'émission du dit rayon laser, ledit groupe (9) comprenant au moins un dispositif de commande et gestion (11), piloté par ledit capteur (10) et configuré de manière à limiter la puissance fournie au dit émetteur (2) de rayon laser quand ladite surface sensible est séparée de la zone à traiter (A) par un écart d'air correspondant à une distance (X) entre la surface sensible du dit capteur de proximité (10) et la zone à traiter (A) supérieure à une valeur préfixée,
où
ledit dispositif de commande et gestion (11) comprend des moyens pour le réglage des grandeurs électriques le long des câbles (12) d'alimentation du dit émetteur (2) de rayon, lesdits moyens étant configurés de manière à limiter les grandeurs électriques d'alimentation de l'émetteur (2) pour limiter l'émission du rayon laser à une valeur inférieure à la valeur de fonctionnement normal, si la distance détectée entre la surface sensible du dit capteur de proximité (10) et la zone à traiter (A) est supérieure à ladite valeur préfixée,
où ledit émetteur (2) est du type à diodes laser,
**caractérisé en ce que**
ledit émetteur (2) est configuré de manière à émettre un rayon ayant une fréquence comprise entre 0,1 et 10 Hz et une énergie comprise entre 0,2 et 25 µJ, si la distance (X) relevée entre la surface sensible du dit capteur de proximité (10) et la zone à traiter est supérieure à ladite valeur préfixée.

2. Appareil selon la revendication 1, **caractérisé en ce que** ledit émetteur (2) est configuré de manière à émettre un rayon ayant une fréquence de 1 Hz et une énergie de 2,5 µJ, si la distance (X) relevée entre la surface sensible du dit capteur de proximité (10) et la zone à traiter est supérieure à la valeur préfixée.

3. Appareil selon la revendication 1, **caractérisé en ce que** ledit capteur de proximité (10) comprend une source lumineuse et une photodiode (13) idoine à relever des photons incidents sur la surface sensible et à envoyer un signal électrique proportionnel à la grandeur de cette détection au dit dispositif de commande et gestion (11), la proximité de l'appareil à la surface à traiter (A) déterminant un réfléchissement élevé de photons émis par la source lumineuse, avec conséquente incidence élevée de ceux-ci sur la photodiode (13), où une distance (X) supérieure à ladite valeur préfixée détermine un faible, si ce n'est nul, réfléchissement des photons émis par la source lumineuse avec conséquente incidence minime de ceux-ci sur ladite photodiode (13).

4. Appareil selon la revendication 3, **caractérisé en ce que** ladite source lumineuse du dit capteur de proximité (10) est constituée du dit émetteur (2) d'un rayon laser où, si l'appareil est allumé, l'émetteur (2) est préprogrammé pour l'activation au fonctionnement à l'intensité d'émission minimale, correspondante à une fréquence comprise entre 0,1 et 10 Hz et une énergie comprise entre 0,2 et 25 µJ.

5. Appareil selon l'une ou plusieurs des revendications précédentes, **caractérisé en ce qu'**il comprend au moins un signal lumineux (14) d'émission du rayon laser.

6. Appareil selon la revendication 5, **caractérisé en ce qu'**il comprend au moins un premier signal lumineux (14) d'indication d'émission du rayon avec intensité minimale et au moins un second signal lumineux (14) d'indication d'émission du rayon avec intensité idoine au traitement de stimulation.

7. Appareil selon l'une ou plusieurs des revendications précédentes, **caractérisé en ce que** ledit dispositif de commande et gestion (11) est du type d'une puce ou d'un circuit intégré disposé le long du dit circuit d'actionnement (3).
